# EUROPÄISCHE PATENTSCHRIFT

(11) **EP 1 012 144 B1**
(45) Veröffentlichungstag und Bekanntmachung des Hinweises auf die Patenterteilung: **12.03.2003**
(21) Anmeldenummer: 98945276.8
(22) Anmeldetag: 21.08.1998
(51) Int. Cl.: C07D 231/22, C07C 239/10

(54) **VERFAHREN ZUR HERSTELLUNG (HETERO)AROMATISCHER HYDROXYLAMINE**
METHOD FOR PRODUCING (HETERO)AROMATIC HYDROXYLAMINES
PROCEDE DE PREPARATION D'HYDROXYLAMINES (HETERO)AROMATIQUES

(30) Priorität: 05.09.1997 DE 19738864; 05.09.1997 DE 19738862
(43) Veröffentlichungstag der Anmeldung: 28.06.2000
(73) Patentinhaber: BASF AKTIENGESELLSCHAFT, 67056 Ludwigshafen (DE)
(72) Erfinder: KLINTZ, Ralf, D-67269 Grünstadt (DE); GÖTZ, Norbert, D-67547 Worms (DE); KEIL, Michael, D-67251 Freinsheim (DE); HEILIG, Manfred, D-67061 Ludwigshafen (DE); WINGERT, Horst, D-68159 Mannheim (DE); VOGELBACHER, Uwe, Josef, D-67071 Ludwigshafen (DE); WAHL, Josef, D-67105 Schifferstadt (DE); WETTERICH, Frank, D-67112 Mutterstadt (DE); DAUN, Gregor, D-69126 Heidelberg (DE)
(74) Vertreter: Fitzner, Ulrich, Dr.
(86) Internationale Anmeldenummer: EP9805332
(87) Internationale Veröffentlichungsnummer: WO99012911

(56) Entgegenhaltungen:
- EP-A- 0 085 890
- DE-A- 4 423 612
- DE-A- 19 502 700
- US-A- 3 544 485
- CHEMICAL ABSTRACTS, vol. 115, no. 17, 28. Oktober 1991 Columbus, Ohio, US; abstract no. 182389, KIJENSKI J. ET AL.: "Selective hydrogenation of aromatic and aliphatic nitro compounds by hydrogen transfer over magnesia" XP002087897 & STUD. SURF. SCI. CATAL., Bd. 59, 1991, Seite 169-176
- KOSAK J R: "HYDROGENATION OF NITROARENES THE HYDROXYLAMINE INTERMEDIATE" CHEMISCHE INDUSTRIE, Bd. 33, 1. Januar 1988, Seiten 135-147, XP000569987

## Beschreibung

Die vorliegende Erfindung betrifft ein Verfahren zur Herstellung (hetero)aromatischer Hydroxylaminderivate der Formel I 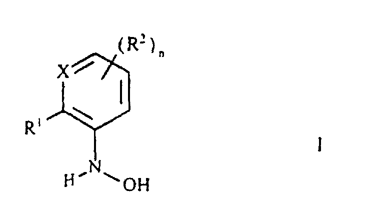 in der die Substituenten, das Ringatom und der Index die folgende Bedeutung haben:
- R¹: Wasserstoff, Halogen, Cyano, C₁-C₄-Alkyl, C₁-C₄-Halogen-alkyl, C₁-C₄-Alkoxy, C₁-C₄-Halogenalkoxy, C₁-C₄-Alkylthio, C₁-C₄-Alkylcarbonyl, C₁-C₄-Dialkylaminocarbonyl, C₁-C₄-Alkylcarbonylamino, C₁-C₄-Alkylcarbonyl-C₁-C₆-alkylamino, C₁-C₄-Alkoxycarbonyl, -CH₂O-N=C(R^{a})-C(R^{b})=N-O-R^{c}, -CH₂-O-N=C(R^{d})-C₁-C₄-Alkyl, oder die Gruppe A-B, wobei
A -O-, -CH₂-, -O-CH₂-, CH₂-O-, -CH₂-O-CO-, -CH=CH-, -CH=N-O-, -CH₂-O-N=C(R^{a})- oder eine Einfachbindung und
B Phenyl, Naphthyl, Pyridinyl, Pyrazinyl, Pyrimidinyl, Pyridazinyl, Pyrazolyl, Imidazolyl, Oxazolyl, Isoxazolyl, Thiazolyl, Isothiazolyl. 1,2,4-Triazolyl, 1,2,3-Triazolyl, Furanyl, Thienyl, Pyrrolyl oder C₃-C₇-Cycloalkyl bedeutet, wobei B mit 1-3 Substituenten Rⁱ substituiert sein kann;
Rⁱ Wasserstoff, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylcarbonyl, C₁-C₄-Alkyl-C(R^{d})=N-O-C₁-C₄-Alkyl, C₁-C₄-Alkoxycarbonyl, C₁-C₄-Alkylaminocarbonyl, C₁-C₄-Dialkylaminocarbonyl, C₁-C₄-Alkylcarbonylamino, C₁-C₄-Alkylcarbonyl-C₁-C₄-alkylamino oder Phenyl, welches selbst durch Halogen oder C₁-C₄-Alkyl substituiert sein kann, bedeuten.
R^{a}, R^{c} Wasserstoff, Halogen, Cyano, C₁-C₄-Alkyl, C₁-C₄-Alkoxy, C₁-C₄-Alkylthio, Cyclopropyl oder Trifluormethyl;
R^{b} C₁-C₄-Alkyl, C₂-C₄-Alkenyl, C₃-C₆-Cycloalkyl, Phenyl, Hetaryl oder Heterocyclyl;
R^{d} unabhängig voneinander Wasserstoff, C₁-C₄-Alkyl, C₂-C₄-Alkenyl, oder C₂-C₄-Alkinyl;
- R²: Halogen, C₁-C₄-Alkyl, C₁-C₄-Halogenalkyl, C₁-C₄-Alkoxycarbonyl;
- X: N oder CH,
- n: 0, 1, 2 oder 3, wobei die Reste R⁴ verschieden sein können, wenn n größer als 1 ist,
durch Hydrierung einer (hetero)aromatischen Nitroverbindung der allgemeinen Formel II, 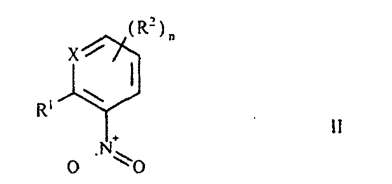 in der R¹, X und R² die vorgenannte Bedeutung haben, in Gegenwart eines Hydrierkatalysators.

In der Literatur (DE-A 2,455,238 und DE-A 2,455,887) ist die Herstellung von Phenylhydroxylaminen durch katalytische Reduktion von Nitroaromaten in Gegenwart von aromatischen Aminen wie Pyridin beschrieben. DE-A 2,357,370, DE-A-1 950 2700 und DE-A 2,327,412 beschreiben ein ähnliches Verfahren unter Zuhilfenahme heterocyclischer Amine wie Piperidin. Ähnliche Verfahren sind ferner an folgender Stelle beschrieben : DE-A-2 327 412, DE-A-2 455 887, DE-A-2 357 370, EP-A 0 147 879, sowie Methoden der Organischen Chemic, Houben-Weyl, Bd. 16a, Teil 1, 1990, Kapitel 1.4.1.1.2. In all diesen Schriften fungiert das Amin gleichzeitig auch als Lösungsmittel. Die nach diesen Verfahren erreichbaren Ausbeuten liegen nach entsprechender Aufarbeitung und Reinigung bei 50-85%; wird in Gegenwart von Pyridin gearbeitet, liegt die Ausbeute in Einzelfällen etwas höher. Das Arbeiten in Pyridin ist jedoch aufgrund der aufwendigeren Aufarbeitung (hoher Siedepunkt, ähnliche Lösungseigenschaften wie das Hydroxylamin) sowie aus Kostengründen wenig wünschenswert.

Ein geeigneteres Verfahren zur Herstellung von (hetero)aromatischen Hydroxylaminen der Formel I der vorliegenden Erfindung wird in DE-A 19,502,700 beschrieben. Hier wird in Gegenwart von speziellen heterocyclischen Aminen, und zwar N-Alkylmorpholinen, gearbeitet, die wie in den bereits erwähnten Schriften auch die Funktion des Lösungsmittels einnehmen. Dieses Verfahren führt zwar zu höheren Ausbeuten an Produkt, erfordert jedoch zur vollständigen Lösung des Ausgangsstoffes hohe Mengen an Alkylmorpholinen und zur Isolierung des Produkts aufgrund von Aduktbildung des Produkts mit dem eingesetzten Alkylmorpholin aufwendige Aufarbeitungsoperationen. Da die Alkylmorpholine bei der Durchführung der Folgestufen erheblich stören, müssen sie mittels Destillation stark abgereichert und in einem zusätzlichen Extraktionsschritt vollständig entfernt werden. Die hohe thermische Belastung führt bei den häufig instabilen Hydroxylaminen zur Beeinträchtigung von Reinheit und Ausbeute.

Der rückgeführte Hydrierkatalysator verliert nach wenigen Zyklen an Aktivität. Die mit der Regenerierung des Katalysators verbundenen Kosten verringern die Wirtschaftlichkeit des Verfahrens.

Schließlich ist aus GB-A 1,092,027 ein Hydrierverfahren zur Herstellung von Cyclohexylhydroxylaminen in Gegenwart von Aminen bekannt. Neben den bereits genannten heterocyclischen Aminen kommt bei diesem Verfahren bevorzugt Cyclohexylamin, ein alicyclisches Amin zum Einsatz. Der Zusatz eines protischen Lösungsmittels wie Ethanol führt in den beschriebenen Beispielen zu einer deutlich niedrigeren Ausbeute. Das andersgeartete Substrat erfordert hinsichtlich der Reaktionstemperatur, des verwendeten Amins und des gegebenenfalls zugesetzten Lösungsmittels spezifische Bedingungen (90°C, Cyclohexylamin, Ethanol), die sich auf eine Hydrierung (hetero)aromatischer Nitroverbindungen nicht übertragen lassen.

Aufgabe der vorliegenden Erfindung war es demnach, ein Verfahren zur Herstellung von N-acylierten (hetero)aromatischen Hydroxylaminen zu finden, das die gezeigten Nachteile nicht aufweist.

Demgemäß wurde das eingangs erwähnte Verfahren gefunden, daß dadurch gekennzeichnet ist, daß man die Hydrierung in einem Gemisch aus einem inerten, aprotischen Lösungsmittel und einem aliphatischen Amin durchführt.

Überraschend bei diesem Verfahren ist, daß gerade aliphatische Amine gute Hydrierergebnisse liefern, weist der Stand der Technik doch explizit auf die Verwendung von (hetero)aromatischen sowie heterocyclischen Aminen hin. Ferner kommt es beim Einsatz von aliphatischen Aminen in geringerem Maße zur Aduktbildung mit dem Hydroxylamin. Ein Großteil des Amins läßt sich dadurch schonend destillativ oder auch extraktiv entfernen.

Überraschend ist weiterhin, daß sich die Hydrierung in Gegenwart von aliphatischen Aminen durch den Zusatz eines apolaren, aprotischen Lösungsmittels dahingehend verbessern läßt, daß die Bildung von unerwünschten Nebenprodukten wie Azoxyverbindungen weitgehend ausbleibt und damit die nach Entfernen des Amins erhaltene rohe Hydriermischung direkt in die Folgestufen eingesetzt werden kann.

Nicht zu erwarten war schließlich, daß der Hydrierkatalysator im erfindungsgemäßen Verfahren eine deutlich höhere Standzeit als in dem mit N-Alkylmorpholinen beschriebenen Verfahren besitzen würde.

Das erfindungsgemäße Verfahren eignet sich vorzugsweise zur Herstellung von (hetero)aromatischen Verbindungen der Formel I, in der
- R¹: -CH₂-O-N=(R^{a})-C(R^{b})=N-O-R⁶, C₁-C₄-Alkyl-CR^{d}=N-O-C₁-C₄-alkyl oder die Gruppe A-B bedeutet, wobei A, B, R^{a}, R^{b}, R^{c} und R^{d} sowie R², X und n die in Anspruch 1 angegebene Bedeutung besitzen.

Insbesondere lassen sich mit dem erfindungsgemäßen Verfahren die in der WO 96/01256 genannten Zwischenprodukte IIIa und Pflanzenschutzmittel IVa herstellen. 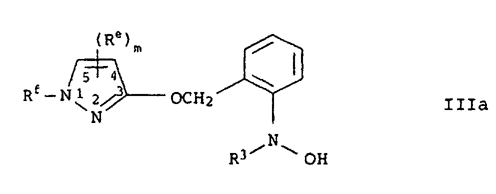 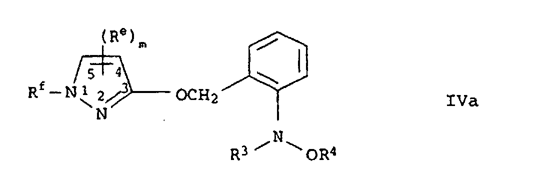 in denen
- R^{f}: C₁-C₆-Alkyl, C₂-C₆-Alkenyl, C₂-C₆-Alkinyl, ggf. subst. gesättigtes oder ein- oder zweifach ungesättigtes Heterocyclyl, ggf. subst. Aryl oder Heteroaryl;
- R^{e}: Halogen, Cyano, C₁-C₄-Alkyl, C₁-C₄-Halogenalkyl, C₁-C₄-Alkoxy, C₁-C₄-Alkylthio und C₁-C₄-Alkoxycarbonyl und
- m: 0, 1, 2 bedeuten
und R³ und R⁴ die in Anspruch 1 gegebene Bedeutung haben (s. Tabellen A und B).

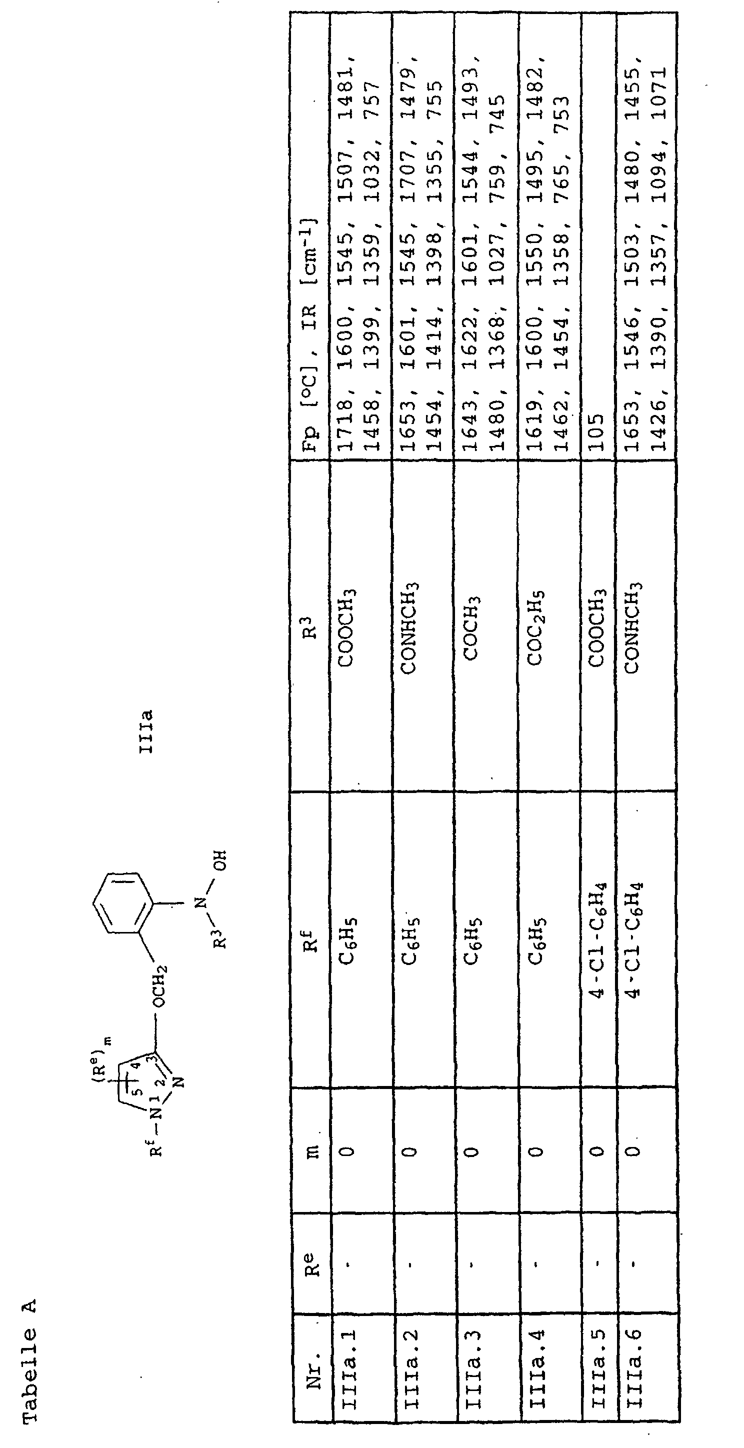

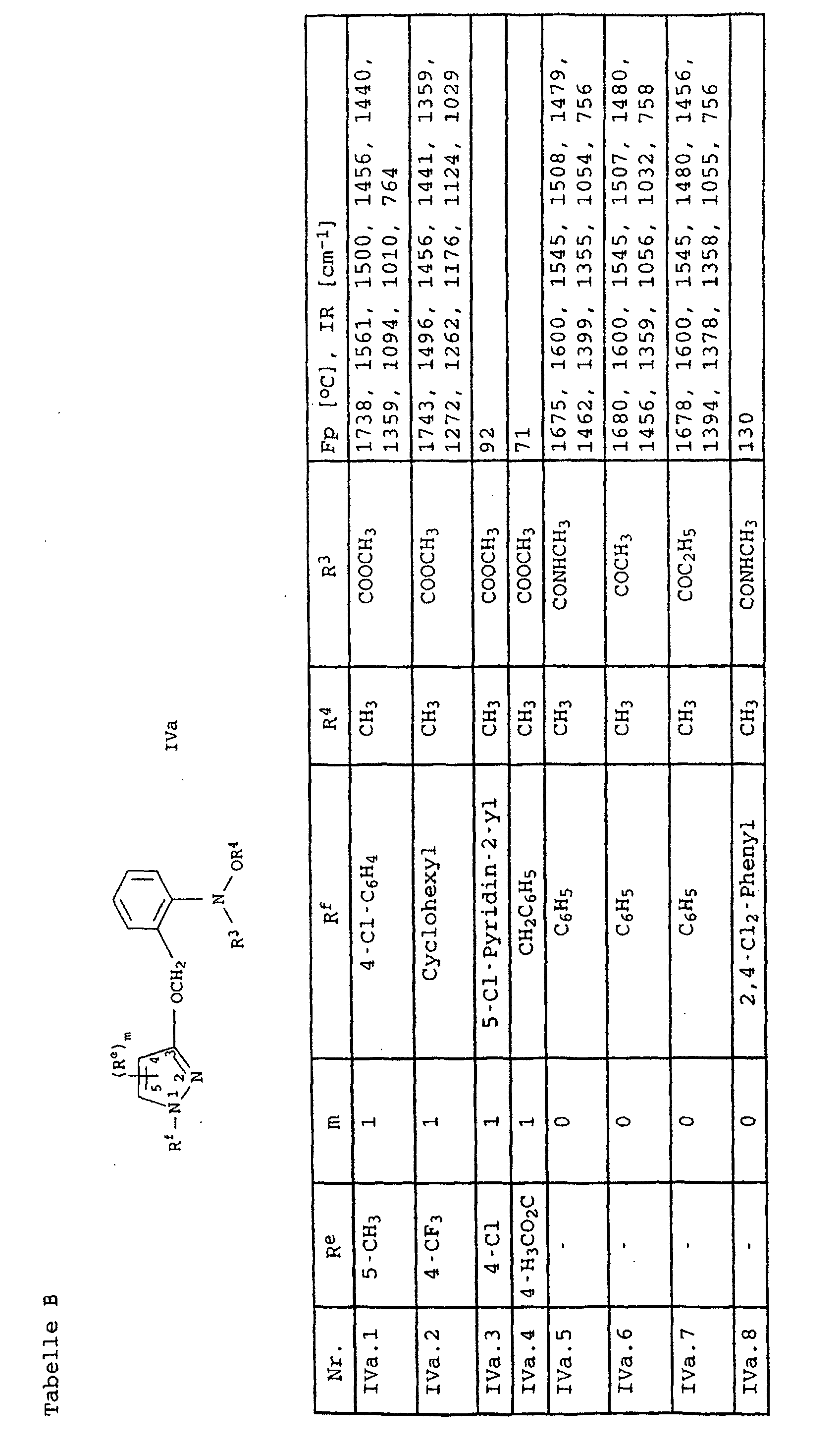

Die Herstellung der Verbindungen III und IV erfolgt vorzugsweise durch N-Acylierung der nach dem erfindungsgemäßen Verfahren hergestellten Hydroxylamine I zu den Verbindungen III und anschließende O-Alkylierung zu den Verbindungen IV (s. Schema 1).

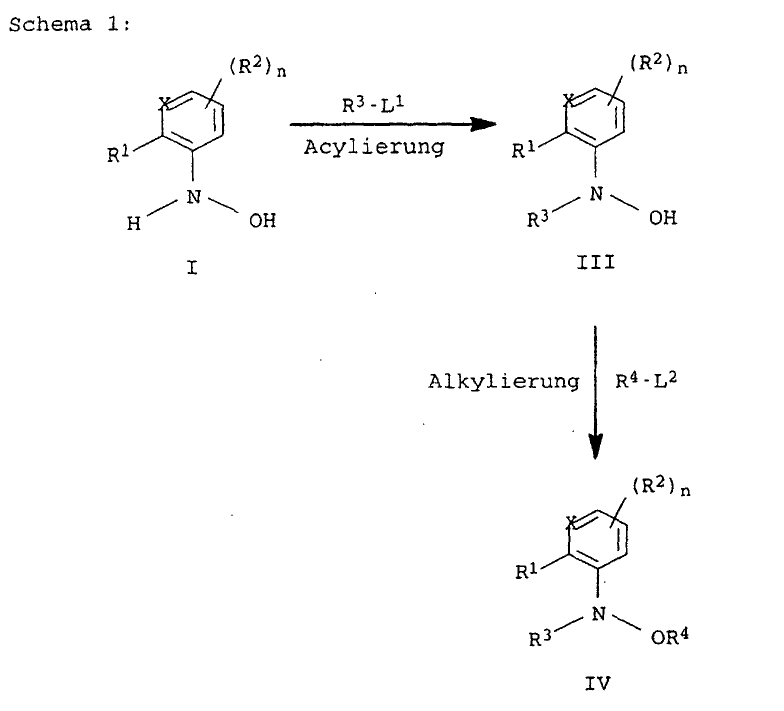

Die Acylierung und Alkylierung werden im Anschluß an die Hydrierung beschrieben.

Im erfindungsgemäßen Verfahren werden primäre, sekundäre oder tertiäre aliphatische Amine eingesetzt. Unter aliphatischen Aminen sind Amine mit einem oder mehreren, geradkettigen oder verzweigten C₁-C₆-Alkylresten zu verstehen. Bevorzugt werden aliphatische Amine eingesetzt, deren Siedepunkt bevorzugt unterhalb dessen des verwendeten inerten, aprotischen Lösungsmittels liegt. Die Amine sind aufgrund des niedrigeren Siedepunktes durch Destillation schonend zu entfernen. Ferner bringt die erwähnte Siedepunktvorgabe mit sich, daß die Amine über eine geringe A1-kylkettenlänge verfügen und damit in der Regel auch eine gute Wasserlöslichkeit besitzen, die wiederum eine einfache Extraktion der Amine durch Wasser zuläßt.

In einer bevorzugten Ausführungsform des Verfahrens werden primäre C₁-C₄-Alkylamine verwendet. Bevorzugt sind n-Propyl-, Isopropyl, n-Butyl- und tert. Butylamin und besonders bevorzugt n-Propylamin. Die C₁-C₄-Alkylamine weisen allesamt einen niedrigen Siedepunkt und eine gute Wasserlöslichkeit auf. Darüberhinaus zeigt sich, daß bei der weiteren Umsetzung der Hydroxylamine I zu N-acylierten Verbindungen III bzw. O-alkylierten Verbindungen IV Restmengen dieser Amine nicht stören, während selbst geringe Mengen von z.B. N-Alkylmorpholinen bei diesen Folgereaktionen zu Ausbeuteverlusten führen. Eine Verwendung der C₁-C₄-Alkylamine ermöglicht damit eine weitere Vereinfachung der Aufarbeitung der Hydriermischung und leistet einen entscheidenden Beitrag zur Prozeßfähigkeit eines Verfahrens zur Herstellung der Verbindungen III und IV.

Als inerte, aprotische Lösungsmittel werden beispielsweise aliphatische oder cyclische Ether wie Tetrahydrofuran oder bevorzugt aliphatische oder aromatische Kohlenwasserstoffe wie Benzol, Toluol oder Chlorbenzol eingesetzt.

In der Regel wird das Amin in einer Konzentration von 0,1 bis 20 Gew.-%, bevorzugt 0,1 bis 15 Gew.-%, im Lösungsmittel eingesetzt. Höhere Konzentrationen sind zwar möglich, bringen aber üblicherweise kaum noch Verbesserungen in Ausbeute und Selektivität und sind daher unwirtschaftlich.

Der gewählte Temperaturbereich für die erfindungsgemäße Hydrierung liegt zwischen -20°C und + 30°C, vorzugsweise zwischen -5 und +10°C. Die Mindesttemperatur ist nur durch den Gefrierpunkt des verwendeten Lösungsmittels bestimmt. Die Höchsttemperatur ist abhängig von der zu hydrierenden Nitroverbindung und den Reaktionsparametern. Um Überhydrierungen zu vermeiden, wird bei der Temperatur, bei der die Hydrierung ausreichend schnell abläuft, ein Druck eingestellt, der zwischen Normaldruck und 10 bar Überdruck liegt. Normalerweise wird der Wasserstoff bei Normaldruck bzw. leicht erhöhtem Druck in den Hydrierreaktor eingegast.

Zur Durchführung des erfindungsgemäßen Verfahrens müssen die Ausgangsstoffe nicht gelöst vorliegen. Die Reaktion führt auch in Suspension zu optimalen Ergebnissen.

Das Amin wird in der Regel in Bezug auf die Nitroverbindung II in einem Molverhältnis von 1 bis 15 eingesetzt.

Im erfindungsgemäßen Verfahren werden handelsübliche Katalysatoren, die beispielsweise Platin oder Palladium auf einem Träger enthalten, oder auch Raney-Nickel oder Raney-Cobalt, eingesetzt.

Sofern in dem Verfahren unter Verwendung von Platin- oder Palladiumkatalysatoren Ausgangsstoffe hydriert werden sollen, die empfindliche Gruppen, wie beispielsweise Halogene oder Benzylether, enthalten, muß der Katalysator gegebenenfalls mit Schwefel oder Selen dotiert sein, um eine ausreichende Selektivität zu erhalten. Der Katalysator kann nach einem Reaktionscyclus abfiltriert und ohne spürbaren Aktivitätsverlust wieder verwendet werden.

Die Verwendung von Platin- oder Palladiumkatalysatoren ist bevorzugt. Der Platin- bzw. Palladiumgehalt des Katalysators ist nicht kritisch und kann in weiten Grenzen variiert werden. Zweckmäßig ist ein Gehalt von 0,1 bis 15 Gew.-%, vorzugsweise von 0,5 bis 10 Gew.-%, bezogen auf das Trägermaterial. Die Menge des eingesetzten Platins oder Palladiums beträgt zwischen 0,001 und 1 Gew.-%, bevorzugt zwischen 0,01 und 0,1 Gew.-%, bezogen auf die Nitroverbindung. In der diskontinuierlichen Hydrierung wird der Katalysator bevorzugt als Pulver eingesetzt. In einer bevorzugten Ausführungsform wird kontinuierlich gearbeitet und als Katalysator Platin oder Palladium auf dem Trägermaterial Kohle verwendet. Andere nicht amphotere Träger, wie Graphit, BaSO₄ oder SiC sind ebenfalls möglich.

Nach Beendigung der Reaktion wird ein Großteil des zugesetzten Amins abdestilliert oder mit Wasser extrahiert. Die Destillation wird vorzugsweise unter Stickstoff, bzw. unter vermindertem Druck durchgeführt. Bei empfindlichen Hydroxylaminen muß auf völligen Ausschluß von Sauerstoff geachtet werden.

Da sich die Handhabung der meist sauerstoffempfindlichen Hydroxylamine z.T. schwierig gestaltet, kann es von Vorteil sein, die Hydroxylamine I unmittelbar nach extraktiver oder destillativer Abtrennung des aliphatischen Amins weiterzuverarbeiten. Bei der destillativen Abtrennung des Amins ist es von Vorteil, wenn das Amin einen niedrigeren Siedepunkt als das Lösungsmittel aufweist. Man erhält eine Lösung des Hydroxylamins im Lösungsmittel, die gleich weiterverarbeitet werden kann.

Zur Herstellung der N-acylierten Verbindungen III und O-alkylierten Verbindungen IV werden die Hydroxylamine I vorzugsweise direkt nach destillativer oder extraktiver Abtrennung des aliphatischen Amins ohne weitere Aufreinigung zu den Verbindungen der Formel III 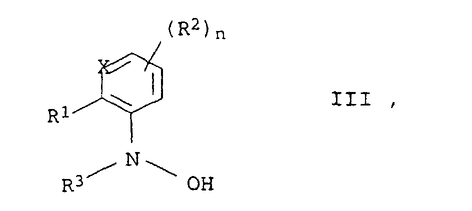 in der die Substituenten R¹ und R², das Ringatom X und Index n die in Anspruch 2 angegebene Bedeutung haben und R³ C₁-C₄-Alkoxycarbonyl, C₁-C₄-Alkylcarbonyl, C₁-C₄-Alkylaminocarbonyl oder Di-(C₁-C₄-alkyl)aminocarbonyl bedeutet, mit Hilfe eines Acylierungsagens R³-L¹, wobei L¹ für eine nukleophile Abgangsgruppe wie Halogenid, Hydroxid, Anhydrid oder Isocyanat steht, N-acyliert und anschließend gegebenenfalls zu den Verbindungen der Formel IV 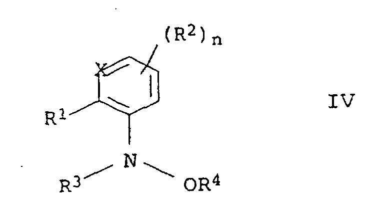 in der die Substituenten R¹, R² und R³, das Ringatom X und Index n die vorgenannte Bedeutung haben und R⁴ für C₁-C₆-Alkyl steht, mit Hilfe eines Alkylierungsmittels R⁴-L², wobei L² für eine nukleophile Abgangsgruppe wie Halogenid, Sulfat oder Sulfonat steht, O-alkyliert.

Die Umsetzung der Hydroxylamine I mit Acylierungsagentien R³-L¹, wobei R³ die vorgenannte Bedeutung und L¹ für eine nukleophile Abgangsgruppe wie z.B. Chlorid steht, erfolgt in der Regel unter alkalischen Bedingungen.

Als Acylierungsagentien kommen beispielsweise Säurechloride, Chlorameisensäure-C₁-C₄-alkylester, wie Chlorameisensäuremethylester, C₁-C₄-Alkancarbonsäurechloride, C₁-C₄-Alkylcarbamidsäurechloride, Di-C₁-C₄-alkylcarbamidsäurechloride, Anhydride oder auch Isocyanate in Betracht.

Außerdem lassen sich als Acylierungsmittel auch die freien Säuren in Verbindung mit einem Kondensationsmittel, wie z.B. Carbonyldiimidazol oder Dicyclohexylcarbondiimid, oder die entsprechenden Anhydride als Acylierungsmittel verwenden.

Die Acylierung erfolgt zweckmäßigerweise in Gegenwart eines inerten organischen Lösungsmittels, das bereits bei der Hydrierung in Verwendung gefunden hat, beispielsweise in einem aprotischen Lösungsmittel wie aliphatischen oder aromatischen Kohlenwasserstoff, wie Toluol, Xylol, Heptan oder Cyclohexan, in einem aliphatischen oder cyclischen Ether, vorzugsweise 1,2-Dimethoxyethan, Tetrahydrofuran oder Dioxan. Es ist auch möglich ein polareres aprotisches Lösungsmittel wie ein aliphatisches Keton, vorzugsweise Aceton, ein Amid, vorzugsweise Dimethylformamid, oder Sulfoxid, vorzugsweise Dimethylsulfoxid, Harnstoffen wie z.B. Tetramethylharnstoff oder 1,3-Dimethyltetrahydro-2(1H)-pyrimidinon, einen Carbonsäureestern, wie z.B. Essigsäureethylester, oder einen halogenierten aliphatischen oder aromatischen Kohlenwasserstoff, wie z.B. Dichlormethan oder Chlorbenzol der Reaktionsmischung zuzusetzen.

In der Regel wird in Gegenwart einer anorganischen Base wie Natrium- oder Kaliumhydroxid, Natriumcarbonat, Natriumhydrogencarbonat, Kaliumcarbonat, Kaliumhydrogencarbonat; eines Amins, wie Triethylamin, Pyridin oder N,N-Diethylanilin; oder eines Alkalialkoholats, wie z.B. Natriummethanolat oder -ethanolat oder Kalium-tert. -butanolat gearbeitet. Die Base ist aber nicht unbedingt erforderlich und kann ggf. durch anderer Säurefänger ersetzt werden, wie z.B. basischer Ionentauscher oder Wasser.

Die Reaktionstemperatur der Acylierung liegt im allgemeinen zwischen 0°C und der Rückflußtemperatur des verwendeten Lösungsmittels, vorzugsweise zwischen 0°C und 50°C.

Die Umsetzung kann auch in einem Zweiphasensystem bestehend aus einer Lösung von Alkali- oder Erdalkalihydroxiden oder -carbonaten in Wasser und einer organischen Phase durchgeführt werden. Als Phasentransferkatalysatoren kommen hierbei beispielsweise Ammoniumhalogenide und -tetrafluoroborate sowie Phosphoniumhalogenide in Betracht. Besonders bevorzugt sind Tetrabutylammoniumchlorid und Benzyltriethylammoniumchlorid.

Die Alkylierung wird üblicherweise in einem inerten Lösungs- oder Verdünnungsmittel durchgeführt, vorzugsweise in Gegenwart einer Base.

Als Lösungs- oder Verdünnungsmittel kommen beispielsweise die in der zuvor beschriebenen Acylierung aufgeführten in Frage.

Zur Alkylierung wird üblicherweise ein Halogenid, vorzugsweise ein Chlorid oder Bromid, ein Sulfat, vorzugsweise Dimethylsulfat, ein Sulfonat, vorzugsweise ein Methansulfonat (Mesylat), Benzolsulfonat, o-Toluolsulfonat (Tosylat) oder p-Brombenzolsulfonat (Brosylat) oder Trifluormethansulfonat (Triflat), oder eine Diazoverbindung eines Alkans verwendet.

Als Base eignen sich anorganische Basen, wie beispielsweise Carbonate, wie Kaliumcarbonat oder Natriumcarbonat, oder Hydrogencarbonate, wie Kalium- oder Natriumhydrogencarbonat, oder Hydroxide, wie Natrium- oder Kaliumhydroxid, Alkalimetallhydride, wie beispielsweise Natriumhydrid oder Kaliumhydrid, oder organische Basen, wie Amine, z.B. Triethylamin, Pyridin oder N,N-Diethylanilin, oder Alkalialkoholate, wie z.B. Natriummethanolat oder -ethanolat oder Kalium-tert.-butanolat.

Vorzugsweise wird das Alkylierungsmittel (beispielsweise Dimethylsulfat) und das N-acylierte Hydroxylamin III vorgelegt und die Base (beispielsweise Kaliumhydroxid) zudosiert.

Die Menge an Base bzw. Alkylierungsmittel liegt vorzugsweise zwischen der halb- und doppelmolaren Menge, bezogen auf die Menge der Verbindung III. Insbesondere bevorzugt werden Base bzw. Alkylierungsmittel im geringen Überschuß eingesetzt.

Im allgemeinen liegt die Reaktionstemperatur bei der Alkylierung zwischen -78°C und der Siedetemperatur des Reaktionsgemisches, bevorzugt zwischen 0 und 100°C und insbesondere bevorzugt zwischen 60 und 90°C.

Wie die Acylierung kann auch die Alkylierung zweiphasig durchgeführt werden. Es können die obengenannten Phasentransferkatalysatoren verwendet werden.

Im Folgenden wird das erfindungsgemäße Verfahren an Hand von Beispielen vorgestellt.

### Beispiel 1

### N-Hydroxy-N-2- [N'-(p-Chlorphenyl)pyrazolyl-3'-oxymethyl]-anilin

a) Hydrierung mit n-Propylamin in Toluol mit Katalysator Pt/C
   In einem 750 ml Kolben mit Gaseinleitungsrohr wurden unter Rühren 60 g (182 mmol) 2-[N-(p-Chlorphenyl)pyrazolyl-3'-oxymethyl]nitrobenzol in 700 ml Toluol vorgelegt. Nach Kühlung auf etwa 5°C wurden 72,8 g (14 Gew.-% bez. auf Toluol) n-Propylamin und 33 g Platin auf Kohle 2,5% zugegeben und das Reaktionsgefäß bei 5°C mit Wasserstoff gespült. Die Hydrierung erfolgte bei einem konstanten Wasserstoffdruck von 100 bar. Die Reaktion war nach HPLC-Analytik nach 2 Std. beendet. Nach Spülen des Reaktionsgefäßes mit Stickstoff wurde das n-Propylamin im Vakuum bei 100-150 mbar und 40-50°C abdestilliert.
   Es wurden 430 ml toluolische Lösung erhalten, die nach HPLC-Analytik 54,8 g N-Hydroxy-N-2-[N'-(p-Chlorphenyl)pyrazol-3'oxymethyl]anilin enthielt (93,4 % Ausbeute).
b) Hydrierung mit n-Propylamin in Toluol mit Katalysator Pt/SiC
   Die Hydrierung wurde unter Verwendung von 1 % Pt auf SiC als Katalysator unter sonst im Beispiel 1a beschriebenen Bedingungen wiederholt. Man erhielt nach der Katalysatorabtrennung das in Beispiel 1a beschriebene Hydroxylamin in einer Ausbeute von 94,2%.
c) Hydrierung mit n-Butylamin in Chlorbenzol
   Eine Lösung von 19 g (57 mmol) 2-[N-(p-Chlorphenyl)pyrazolyl-3'-oxymethyl]nitrobenzol in 500 ml Chlorbenzol wurde mit 42 g (0,57 mol) n-Butylamin und 1,9 g Pt/C 5% (F 105 XRS/W der Fa. Degussa) versetzt. Nach Kühlung auf etwa 5°C und Spülen mit Stickstoff und Wasserstoff wurde bei 5 bis 7 °C bei einem konstanten Wasserstoffdruck von 100 mbar hydriert. Die Reaktion war nach HPLC-Analytik nach 35 min beendet. Nach Spülen des Reaktionsgefäßes mit Stickstoff wurde der Katalysator abfiltriert und die Reaktionslösung im Vakuum bei 40°C und 30-400 mbar zur Trockne eingeengt. Es wurden 16,7 g Rückstand erhalten, der nach HPLC-Analytik 94,4 Gew.-% der Titelverbindung enthielt, was 87 % Ausbeute entspricht.

### Beispiel 2

Herstellung von N-Hydroxy-N-(2-[N'-(p-Chlorphenyl)pyrazolyl-3'oxymethyl]-phenyl)-carbamidsäuremethylester
i) N-Hydroxy-N-2-[N'-(p-Chlorphenyl)pyrazolyl-3'-oxymethyl]-anilin
   entspricht Beispiel 1a.
ii) N-Hydroxy-N-(2-[N'-(p-Chlorphenyl)pyrazolyl-3'-oxymethyl]-phenyl)-carbamidsäuremethylester
   Zu der aus der Destillation erhaltenen toluolischen Lösung wurden bei 30°C unter Stickstoff 51 g Toluol und 33 g Wasser gegeben. Zu der erhaltenen Emulsion wurden unter intensivem Rühren innerhalb von 2 Std. 19 g (0,19 mol) Chlorameisensäuremethylester gegeben. Nach weiteren 2,5 Std. Rühren bei 30°C wurde bei 15°C der Niederschlag abfiltriert und im Vakuum bei 40°C getrocknet. Man erhielt 59,7 g der Titelverbindung (nach ¹H-NMR >95 Gew.-%ig), was einer Ausbeute von 88 % über beide Stufen entspricht.

### Beispiel 3 (Vergleichsbeispiel)

Es wurden folgende Vergleichsversuche zur Herstellung von N-Hydroxy-N-2-[N'-(p-Chlorphenyl)pyrazolyl-3'-oxymethyl]-anilin durchgeführt:
a) Umsetzung in primärem Amin als Lösungsmittel
   Eine Lösung von 15 g (45 mmol) 2-[N-(p-Chlorphenyl)pyrazolyl-3-oxymethyl]nitrobenzol in 261 g n-Propylamin wurde nach Zusatz von 1,2 g Pt/C 5% (F 105 XRS/W 55 der Fa. Degussa) und Inertisieren mit Stickstoff und Spülen mit Wasserstoff bei 0°C wurde bei 0 bis 5°C und 100 mbar Überdruck während 25 min die theoretisch zur vollständigen Umsetzung nötige Wasserstoffmenge eingeleitet. Die HPLC-Analyse der Reaktionslösung ergab neben Spuren des Ausgangsmaterials und etwa 55 Gew.-% des gewünschten Produkts noch etwa 22 Gew.-% der Azoxyverbindung der Molmasse 614.
b) Umsetzung mit Lösungsmittel ohne Amin
   15 g (45 mmol) 2-[N-(p-Chlorphenyl)pyrazolyl-3-oximethyl]nitrobenzol wurden in 350 ml Toluol gelöst und mit 1,2 g Pt/C 5% (F 105 XRS/W 55 der Fa. Degussa) versetzt. Nach Inertisieren und Spülen mit Wasserstoff bei 0°C wurde bei 0 bis 5°C und 100 mbar Überdruck während 3 h hydriert. Nach dieser Zeit wurden noch etwa 90 % Ausgangsmaterial und etwa 10% des gewünschten Produkts nachgewiesen. Der Versuch wurde abgebrochen.
c) Umsetzung mit N-Methylmorpholin (analog DE-A 195 02 700)
   Zu einer Lösung von 120 g 2-[N-(p-Chlorphenyl)pyrazolyl-3-oxymethyl]-nitrobenzol und 10 g Aktivkohle in 2,2 l N-Methylmorpholin wurden bei etwa 20°C 10 g Pt/C Kontakt (F 105 XRS/W 55 der Fa. Degussa) gegeben. Nach Spülen mit Stickstoff und Wasserstoff wurde bei 20 bis 30°C und einem konstanten Wasserstoffdruck von 100 mbar ca. 2,5 h hydriert. Anschließend wurde der Katalysator abfiltriert und das Reaktionsgemisch bei 50°C und 20 mbar im Vakuum eingeengt. Zur Verdrängung der verbleibenden N-Methylmorpholinmenge wurden ca 700 ml Benzin 186-213 zugegeben und erneut bei 50 - 60°C und 0,5 mbar eingeengt. Das erhaltene Produkt wurde in 85 ml Methanol gelöst und auf 0°C abgekühlt. Der erhaltene Niederschlag wurde abgesaugt und bei 30°C im Vakuum getrocknet. Man erhielt 92,7 g des gewünschten Produkts (nach HPLC 95 Gew.-%ig), was einer Ausbeute von 81 % entspricht.

### Beispiel 4 (Vergleichsbeispiel analog DE-A 19,502,700)

Herstellung von N-Hydroxy-N-(2-[N'-(p-Chlorphenyl)pyrazolyl-3'oxymethyl]-phenyl)-carbamidsäuremethylester
i) N-Hydroxy-N-2-[N'-(p-Chlorphenyl)pyrazolyl-3'-oxymethyl]-anilin
   entspricht Beispiel 3c.
ii) N-Hydroxy-N-(2-[N'-(p-Chlorphenyl)pyrazolyl-3'-oxymethyl]-phenyl)-carbamidsäuremethylester

Die Umsetzung wurde entsprechend Beispiel 2ii) durchgeführt und lieferte 93 % Ausbeute. Über beide Stufen wurde damit eine Ausbeute von 75 % erzielt.

### Beispiel 5

### N-(2-Tolyl)hydroxylamin

### Hydrierung in Gegenwart von n-Propylamin

In einem 1,5-1-Hydrierkolben mit Gaseinleitungsrohr wurden unter Rühren 41,1 g (0,3 mol) o-Nitrotoluol in 600 ml Toluol mit 5,1 g Aktivkohle vorgelegt. Nach Kühlung auf etwa 5 bis 8°C wurden 67,4 g (1,1 mol) n-Propylamin und 3 g Platin auf Kohle 5% (CF 105 XRS der Fa. Degussa) zugegeben und das Reaktionsgefäß bei 5°C mit Stickstoff, dann mit Wasserstoff gespült. Die Hydrierung erfolgte bei einem konstanten Wasserstoffdruck von 100 mbar Überdruck. Die Reaktion war nach HPLC-Analytik nach 100 min beendet.

Nach Spülen des Reaktionsgefäßes mit Stickstoff wurde das Amin bei 60°C abdestilliert. Nach HPLC-Analyse liegt das N-(2-Tolyl)-hydroxylamin in einer Reinheit von 98-99 % in toluolischer Lösung vor.

### Beispiel 6 (Vergleichsbeispiel)

### N-(2-Tolyl)hydroxylamin

### Hydrierung in Gegenwart von M-Methylmorpholin

Eine Suspension von 411 g (3 mol) 2-Nitrotoluol und 15 g Platin auf Aktivkohle (F 105 XRS/W 5% der Fa. Degussa) in 2,8 l 4-Methylmorpholin wurde bei 0°C mit Stickstoff, dann mit Wasserstoff gespült. die Reaktion wurde bei 100 mbar Überdruck durchgeführt. Nach 13 Std. war die Umsetzung beendet. Nach Spülen mit Stickstoff und Abfiltrieren des Katalysators wurde das Lösungsmittel bei 45 bis 50°C im Vakuum weitestgehend abdestilliert. Der Rückstand wurde in 1 l Methylenchlorid/Wasser (1:1) aufgenommen und die wäßrige Phase nach Ansäuern mit Salzsäure mit Methylenchlorid extrahiert. Nach Trocknen der organischen Phasen und Abdestillieren des Lösungsmittels wurde der Rückstand in Pentan digeriert, abfiltriert und gewaschen. Man erhielt 305 g der Titelverbindung als (nach HPLC-Analyse) 89 Gew.-%iges Produkt.

### Beispiel 7

Die überraschend bessere Eignung der Reaktionslösung mit Resten des Aminzusatzes aus dem erfindungsgemäßen Verfahren zu direkten weiteren Umsetzung des Reaktionsproduktes mit Chlorameisensäuremethylester ließ sich durch folgende Vergleichsversuche zeigen:

### N-Hydroxy-N-(2-N'-(p-Chlorphenyl)pyrazolyl-3'-oxymethyl]phenyl)-carbamidsäuremethylester

I) Erfindungsgemäß unter Anwesenheit von n-Propylamin:
   Zu einer Suspension von 10 g N-Hydroxy-N-(2-[N'-(p-Chlorphenyl)pyrazolyl-3'-oxymethyl]-anilin in 140 ml Toluol wurden 1,4 ml n-Propylamin gegeben. Anschließend wurden 3,13 g Natriumhydrogencarbonat zugesetzt und innerhalb von 10 min 3,1 g Chlorameisensäuremethylester zugegeben. Nach etwa 14 h Rühren bei etwa 20°C wurde der Feststoff abgesaugt und nach Waschen mit Wasser Vakuum getrocknet. Man erhielt 10,8 g der Titelverbindung (nach ¹H-NMR >95 Gew.-%ig), was 94 % Ausbeute entspricht.
II) Analog DE-A 195 02 700 unter Anwesenheit von N-Methylmorpholin:
   Der Versuch wurde unter Zusatz von 1,4 ml N-Methylmorpholin unter sonst im Beispiel 7I beschriebenen Bedingungen wiederholt. Nach Fällung, Waschen und Trocknung des Niederschlages erhielt man 8 g Titelverbindung (nach ¹H-NMR >95 Gew.-%ig), was 69 % Ausbeute entspricht.

Die Acylierung zeigt sich überraschenderweise unempfindlich gegenüber der Anwesenheit von ca. 10 Gew.-% n-Propylamin, während der Zusatz entsprechender Mengen N-Methylmorpholin zu einer deutlichen Ausbeuteverringerung führt.

## Patentansprüche

1. Verfahren zur Herstellung (hetero)aromatischer Hydroxylaminderivate der Formel I 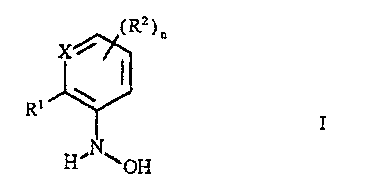 in der die Substituenten, das Ringatom und der Index die folgende Bedeutung haben:
R¹ Wasserstoff, Halogen, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylcarbonyl, C₁-C₄-Dialkylaminocarbonyl, C₂-C₄-Alkylcarbonylamino, C₁-C₄-Alkylcarbonyl-C₁-C₆-alkylamino, C₁-C₄-Alkoxycarbonyl, -CH₂O-N=C(R^{a})-C(R^{b})=N-O-R^{c}, -CH₂-O-N=C(R^{d})-C₁-C₄-Alkyl, oder die Gruppe A-B, wobei
A -O-, -CH₂-, -O-CH₂-, CH₂-O-, -CH₂-O-CO-, -CH=CH-, -CH=N-O-, -CH₂-O-N=C(R^{a})- oder eine Einfachbindung und
B Phenyl, Naphthyl, Pyridinyl, Pyrazinyl, Pyrimidinyl, Pyridazinyl, Pyrazolyl, Imidazolyl, Oxazolyl, Isoxazolyl, Thiazolyl, Isothiazolyl, 1,2,4-Triazolyl, 1,2,3-Triazolyl, Furanyl, Thienyl, Pyrrolyl oder C₃-C₇-Cycloalkyl bedeutet, wobei B mit 1-3 Substituenten Rⁱ substituiert sein kann;
Rⁱ Wasserstoff, Halogen, Cyano, C₁-C₄-Alkyl, C₁-C₄-Halogenalkyl, C₁-C₄-Alkoxy, C₁-C₄-Halo-genalkoxy, C₁-C₄-Alkylthio, C₁-C₄-Alkylcarbonyl, C₁-C₄-Alkyl-C(R^{d})=N-O-C₁-C₄-alkylen, C₁-C₄-Alkoxycarbonyl, C₁-C₄-Alkylaminocarbonyl, C₁-C₄-Dialkylaminocarbonyl, C₁-C₄-Alkylcarbonylamino, C₁-C₄-Alkylcarbonyl-C₁-C₄-alkylamino oder Phenyl, welches selbst durch Halogen oder C₁-C₄-Alkyl substituiert sein kann, bedeuten.
R^{a}, R^{c} Wasserstoff, Halogen, Cyano, C₁-C₄-Alkyl, C₁-C₄-Alkoxy, C₁-C₄-Alkylthio, Cyclopropyl oder Trifluormethyl;
R^{b} C₁-C₄-Alkyl, C₂-C₄-Alkenyl, C₃-C₆-Cycloalkyl, Phenyl, Hetaryl oder Heterocyclyl;
R^{d} unabhängig voneinander Wasserstoff, C₁-C₄-Alkyl, C₂-C₄-Alkenyl, oder C₂-C₄-Alkinyl;
R² Halogen, C₁-C₄-Alkyl, C₁-C₄-Halogenalkyl, C₁-C₄-Alkoxycarbonyl;
X N oder CH,
n 0, 1, 2 oder 3, wobei die Reste R² verschieden sein können, wenn n größer als 1 ist,
durch Hydrierung einer (hetero)aromatischen Nitroverbindung der allgemeinen Formel II, 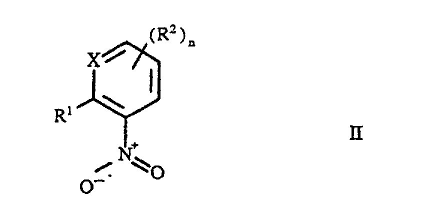 in der R¹, X und R² die vorgenannte Bedeutung haben, in Gegenwart eines Hydrierkatalysators, **dadurch gekennzeichnet, daß** man die Hydrierung in einem Gemisch bestehend aus einem inerten, aprotischen Lösungsmittel und einem C₁-C₄-Alkylamin durchführt.

2. Verfahren gemäß Anspruch 1 zur Herstellung von (hetero)aromatischen Verbindungen der Formel I, in der
R¹ -CH₂-O-N=C(R^{a})-C(R^{b})=N-O-R^{c}, C₁-C₄-Alkyl-CR^{d}=N-O-CH₂ oder die Gruppe A-B bedeutet, wobei A, B, R^{a}, R^{b}, R^{c} und R^{d} sowie R², X und n die in Anspruch 1 angegebene Bedeutung besitzen.

3. Verfahren gemäß einem der Ansprüche 1 oder 2, **dadurch gekennzeichnet, daß** das C₁-C₄-Alkylamin einen Siedepunkt unter dem des Lösungsmittels aufweist.

4. Verfahren gemäß einem der Ansprüche 1, 2 oder 3, **dadurch gekennzeichnet, daß** die Hydrierung in einem Gemisch aus einem aromatischen oder aliphatischen Kohlenwasserstoff und einem aliphatischen Amin durchgeführt wird.

5. Verfahren gemäß einem der Ansprüche 1, 2 oder 3, **dadurch gekennzeichnet, daß** als Hydrierkatalysator Palladium oder Platin ggf. in Gegenwart eines Aktivkohleträgers, verwendet wird.

6. Verfahren gemäß einem der Ansprüche 1, 2 oder 3, **dadurch gekennzeichnet, daß** als Hydrierkatalysator Raney-Nickel oder Raney-Cobalt verwendet wird.

7. Verfahren gemäß einem der Ansprüche 1 2 oder 3, **dadurch gekennzeichnet, daß** das aliphatische Amin in Bezug auf die Nitroverbindung II in einem Molverhältnis von 1 bis 15 eingesetzt wird.

8. Verfahren gemäß einem der Ansprüche 1, 2 oder 3, **dadurch gekennzeichnet, daß** die Konzentration des aliphatischen Amins im Lösungsmittel 0,1 bis 20 Gew.-% beträgt.

9. Verfahren gemäß einem der Ansprüche 1, 2 oder 3, **dadurch gekennzeichnet, daß** nach beendeter Hydrierung das Amin destillativ oder extraktiv entfernt wird und das so erhaltene Hydroxylamin I ohne weitere Aufreinigung zu den Verbindungen der Formel III 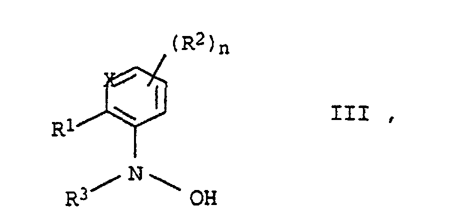 in der die Substituenten R¹ und R², das Ringatom X und Index n die in Anspruch 2 angegebene Bedeutung haben und R³ C₁-C₄-Alkoxycarbonyl, C₁-C₄-Alkylcarbonyl, C₁-C₄-Alkylaminocarbonyl oder Di-(C₁-C₄-alkyl)aminocarbonyl bedeutet, mit Hilfe eines Acylierungsagens R³-L¹, wobei L¹ für eine nukleophile Abgangsgruppe wie Halogenid, Hydroxid, Anhydrid oder Isocyanat steht, N-acyliert und anschließend gegebenenfalls zu den Verbindungen der Formel IV 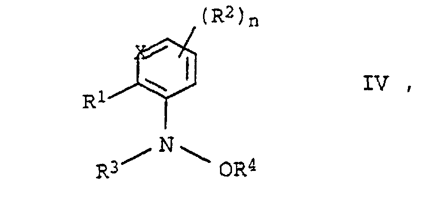 in der die Substituenten R¹, R² und R³, das Ringatom X und Index n die die vorgenannte Bedeutung haben und R⁴ für C₁-C₆-Alkyl steht, mit Hilfe eines Alkylierungsmittels R⁴-L², wobei L² für eine nukleophile Abgangsgruppe wie Halogenid, Sulfat oder Sulfonat steht, O-alkyliert.

## Claims

1. A process for the preparation of N-acylated (hetero)aromatic hydroxylamine derivates of the formula I 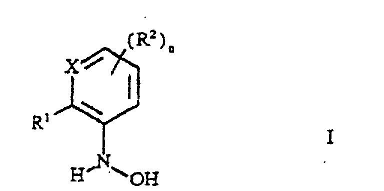 where:
R¹ is hydrogen, halogen, cyano, C₁-C₄-alkyl, C₁-C₄-haloalkyl, C₁-C₄-alkoxy, C₁-C₄-haloalkoxy, C₁-C₄-alkylthio, C₁-C₄-alkylcarbonyl, C₁-C₄-di-alkylaminocarbonyl, C₂-C₄-alkylcarbonylamino, C₁-C₄-alkylcarbonyl-C₁-C₆-alkylamino, C₁-C₄-alkoxycarbonyl, -CH₂O-N=C (R^{a})-C(R^{b})=N-O-R^{c}, -CH₂-O-N=C(R^{d})-C₁-C₄-alkyl, or a group A-B, where
A is -O-, -CH₂-, -O-CH₂-, -CH₂-O-, -CH₂-O-CO-, -CH=CH-, -CH=N-O-, -CH₂-O-N=C(R^{a})- or a single bond,
B is phenyl, naphthyl, pyridyl, pyrazinyl, pyrimidinyl, pyridazinyl, pyrazolyl, imidazolyl, oxazolyl, isoxazolyl, thiazolyl, isothiazolyl, 1,2,4-triazolyl, 1,2,3-triazolyl, furanyl, thienyl, pyrrolyl or C₃-C₇-cycloalkyl, where B may be substituted by 1-3 substituents Rⁱ,
Rⁱ is hydrogen, halogen, cyano, C₁-C₄-alkyl, C₁-C₄-haloalkyl, C₁-C₄-alkoxy, C₁-C₄-halo-alkoxy, C₁-C₄-alkylthio, C₁-C₄-alkylcarbonyl, C₁-C₄-alkyl-C(R^{d})=N-O-C₁-C₄-alkylene [sic], C₁-C₄-alkoxycarbonyl, C₁-C₄-alkylaminocarbonyl, C₁-C₄-dialkylaminocarbonyl, C₁-C₄-alkylcarbonylamino, C₁-C₄-alkylcarbonyl-C₁-C₄-alkylamino or phenyl which itself may be substituted by halogen or C₁-C₄-alkyl,
R^{a} and R^{c} are each hydrogen, halogen, cyano, C₁-C₄-alkyl, C₁-C₄-alkoxy, C₁-C₄-alkylthio, cyclopropyl or trifluoromethyl,
R^{b} is C₁-C₄-alkyl, C₂-C₄-alkenyl, C₃-C₆-cycloalkyl, phenyl, hetaryl or heterocyclyl,
R^{d} independently of one another, are hydrogen, C₁-C₄-alkyl, C₂-C₄-alkenyl, or C₂-C₄-alkynyl,
R² is halogen, C₁-C₄-alkyl, C₁-C₄-haloalkyl or C₁-C₄-alkoxycarbonyl,
X is N or CH and
n is 0, 1, 2 or 3, it being possible for the radicals R² to be different when n is greater than 1,
by hydrogenating a (hetero)aromatic nitro compound of the formula II 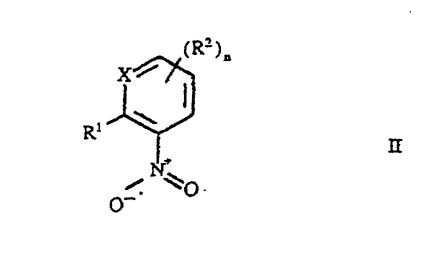 where R¹, X and R² have the abovementioned meanings, in the presence of a hydrogenation catalyst, wherein the hydrogenation is carried out in a mixture consisting of an inert, aprotic solvent and a C₁-C₄-alkylamine.

2. A process as claimed in claim 1 for the preparation of (hetero)aromatic compounds of the formula I, where
R¹ is -CH₂-O-N=C(R^{a})-C(R^{b})=N-O-R^{c}, C₁-C₄-alkyl-CR^{d}=N-O-CH₂ or the group A-B where A, B, R^{a}, R^{b}, R^{c} and R^{d} and R², X and n have the meanings stated in claim 1.

3. A process as claimed in either of claims 1 and 2, wherein the C₁-C₄-alkylamine has a boiling point below that of the solvent.

4. A process as claimed in any of claims 1, 2 and 3, wherein the hydrogenation is carried out in a mixture of an aromatic or aliphatic hydrocarbon and an aliphatic amine.

5. A process as claimed in any of claims 1, 2 and 3, wherein palladium or platinum, in the presence or absence of an active carbon carrier, is used as the hydrogenation catalyst.

6. A process as claimed in any of claims 1, 2 and 3, wherein Raney nickel or Raney cobalt is used as the hydrogenation catalyst.

7. A process as claimed in any of claims 1, 2 and 3, wherein the aliphatic amine is used in a molar ratio of from 1 to 15, based on the nitro compound II.

8. A process as claimed in any of claims 1, 2 and 3, wherein the concentration of the aliphatic amine in the solvent is from 0.1 to 20% by weight.

9. A process as claimed in any of claims 1, 2 and 3, wherein, after hydrogenation is complete, the amine is removed by distillation or extraction and the hydroxylamine I thus obtained, without further purification, is N-acylated to give the compounds of the formula III 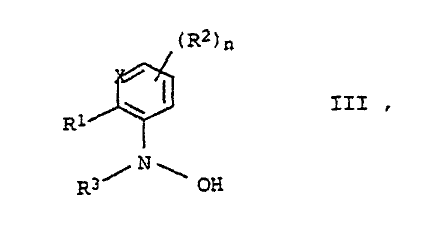 where R¹ and R², the ring atom X and n have the meanings stated in claim 2 and R³ is C₁-C₄-alkoxycarbonyl, C₁-C₄-alkylcarbonyl, C₁-C₄-alkylaminocarbonyl or di(C₁-C₄-alkyl)aminocarbonyl, with the aid of an acylating agent R³-L¹, where L¹ is a nucleophilic leaving group, such as halide, hydroxide, anhydride or isocyanate, and then, if required, O-alkylated to give the compounds of the formula IV 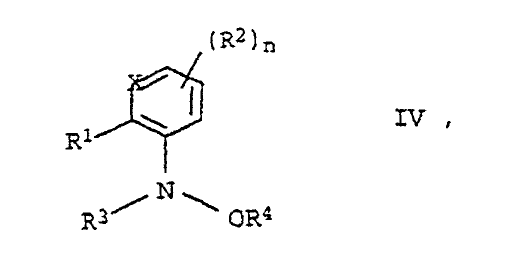 where R¹, R² and R³, the ring atom X and n have the abovementioned meanings and R⁴ is C₁-C₆-alkyl, with the aid of an alkylating agent R⁴-L², where L² is a nucleophilic leaving group, such as halide, sulfate or sulfonate.

## Revendications

1. Procédé pour la préparation de dérivés (hétéro)aromatiques d'hydroxylamine de formule I 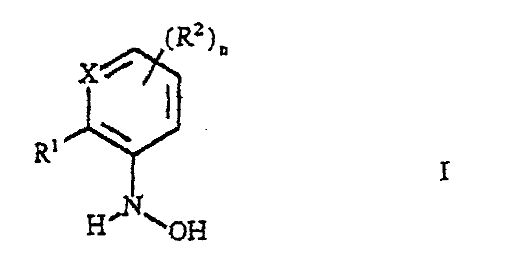 dans laquelle les substituants, l'atome dans le cycle et l'indice ont les significations suivantes :
R¹ représente un atome d'hydrogène ou d'halogène ou un groupe cyano, alkyle en C₁-C₄, halogénoalkyle en C₁-C₄, alcoxy en C₁-C₄, halogénoalcoxy en C₁-C₄, alkyl(C₁-C₄)thio, alkyl(C₁-C₄)carbonyle, dialkyl(C₁-C₄)aminocarbonyle, alkyl(C₂-C₄)carbonylamino, alkyl(C₁-C₄)carbonyl-alkyl(C₁-C₆)amino, alcoxy(C₁-C₄)carbonyle, -CH₂O-N=C(R^{a})-C(R^{b})=N-O-R^{c}, -CH₂-O-N=C(R^{d})-alkyl(C₁-C₄) ou le groupe A-B,
A représentant -O-, -CH₂-, -O-CH₂-, -CH₂-O-, -CH₂-O-CO-, -CH=CH-, -CH=N-O-, -CH₂-O-N=C(R^{a})- ou une liaison simple et
B représentant un groupe phényle, naphtyle, pyridinyle, pyrazinyle, pyrimidinyle, pyridazinyle, pyrazolyle, imidazolyle, oxazolyle, isoxazolyle, thiazolyle, isothiazolyle, 1,2,4-triazolyle, 1,2,3-triazolyle, furannyle, thiényle, pyrrolyle ou cycloalkyle en C₃-C₇, B pouvant porter 1-3 substituants Rⁱ ;
Rⁱ représentant un atome d'hydrogène ou d'halogène ou un groupe cyano, alkyle en C₁-C₄, halogénoalkyle en C₁-C₄, alcoxy en C₁-C₄, halogénoalcoxy en C₁-C₄, alkyl(C₁-C₄)thio, alkyl(C₁-C₄)carbonyle, alkyl(C₁-C₄)-C(R^{d})=N-O-alkyle(C₁-C₄), alcoxy(C₁-C₄)carbonyle, alkyl(C₁-C₄)aminocarbonyle, dialkyl(C₁-C₄)aminocarbonyle, alkyl(C₁-C₄)carbonylamino, alkyl(C₁-C₄)carbonyl-alkyl(C₁-C₄)amino ou phényle, qui peut lui-même être substitué par un atome d'halogène ou par un groupe alkyle en C₁-C₄ ;
R^{a}, R^{c} représentant un atome d'hydrogène ou d'halogène ou un groupe cyano, alkyle en C₁-C₄, alcoxy en C₁-C₄, alkyl(C₁-C₄)thio, cyciopropyle ou trifluorométhyle ;
R^{b} représentant un groupe alkyle en C₁-C₄, alcényle en C₂-C₄, cycloalkyle en C₃-C₆, phényle, hétéroaryle ou hétérocyclyle
R^{d} représentant, indépendamment les uns des autres, un atome d'hydrogène, un groupe alkyle en C₁-C₄, alcényle en C₂-C₄ ou alcynyle en C₂-C₄ ;
R² représente un atome d'halogène ou un groupe alkyle en C₁-C₄, hatogénoalkyle en C₁-C₄, alcoxy(C₁-C₄)carbonyle;
X représente N ou CH ;
n est 0, 1, 2 ou 3, les radicaux R⁴ pouvant être différents lorsque n est supérieur à 1,
par hydrogénation d'un composé nitré (héréro)aromatique de formule générale II 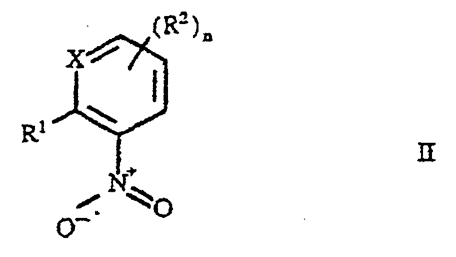 dans laquelle R¹, X et R² ont les significations précitées, en présence d'un catalyseur d'hydrogénation, **caractérisé en ce qu'**on effectue l'hydrogénation dans un mélange constitué d'un solvant aprotique inerte et d'une alkyl(C₁-C₄)amine.

2. Procédé selon la revendication 1, pour la préparation de composés (hétéro)aromatiques de formule I, dans laquelle
R¹ représente un groupe -CH₂-O-N=(R^{a})-C(R^{b})=N-O-R^{c}, alkyl(C₁-C₄)CR^{d}=N-O-CH₂ ou le groupe A-B, A, B, R^{a}, R^{b}, R^{c}, R^{d} ainsi que R², X et n ayant les significations indiquées dans la revendication 1.

3. Procédé selon la revendication 1 ou 2, **caractérisé en ce que** l'alkyl(C₁-C₄)amine a un point d'ébullition inférieur à celui du solvant.

4. Procédé selon l'une quelconque des revendications 1, 2 et 3, **caractérisé en ce que** l'hydrogénation est effectuée dans un mélange d'un hydrocarbure aromatique ou aliphatique et d'une amine aliphatique.

5. Procédé selon l'une quelconque des revendications 1, 2 et 3, **caractérisé en ce qu'**on utilise comme catalyseur d'hydrogénation du palladium ou du platine, éventuellement en présence d'un support constitué de charbon actif.

6. Procédé selon l'une quelconque des revendications 1, 2 et 3, **caractérisé en ce qu'**on utilise comme catalyseur d'hydrogénation du nickel de Raney ou du cobalt de Raney.

7. Procédé selon l'une quelconque des revendications 1, 2 et 3, **caractérisé en ce qu'**on utilise l'amine aliphatique en un rapport molaire de 1 à 15, relativement au composé nitré II.

8. Procédé selon l'une quelconque des revendications 1, 2 et 3, **caractérisé en ce que** la concentration de l'amine aliphatique dans le solvant va de 0,1 à 20 % en poids.

9. Procédé selon l'une quelconque des revendications 1, 2 et 3, **caractérisé en ce que**, une fois l'hydrogénation terminée, on élimine l'amine par distillation ou extraction, et l'hydroxylamine I ainsi obtenue est N-acylée, sans être purifiée davantage, en les composés de formule III 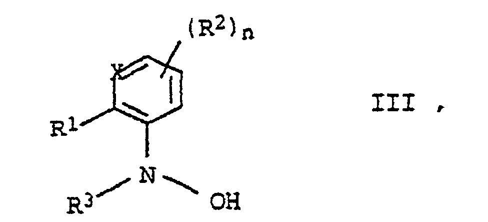 dans laquelle les substituants R¹ et R², l'atome X présent dans le cycle et l'indice n ont les significations indiquées dans la revendication 2, et R³ représente un groupe alcoxy(C₁-C₄)carbonyle, alkyl(C₁-C₄)carbonyle, alkyl(C₁-C₄)aminocarbonyle ou dialkyl(C₁-C₄)aminocarbonyle, à l'aide d'un réactif d'acylation R³-L¹, L¹ représentant un groupe partant nucléophile tel qu'un halogénure, un hydroxyde, un anhydride ou un isocyanate, et ensuite éventuellement O-alkylée en les composés de formule IV 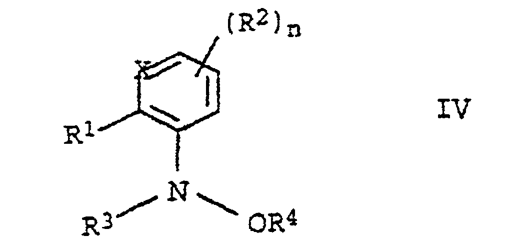 dans laquelle les substituants R¹, R² et R³, l'atome X présent dans le cycle et l'indice n ont les significations indiquées précédemment et R⁴ représente un groupe alkyle en C₁-C₆, à l'aide d'un agent d'alkylation R⁴-L², L² représentant un groupe partant nucléophile tel qu'un halogénure, un sulfate ou un sulfonate.
